# EUROPEAN PATENT APPLICATION

(11) **EP 3 156 009 A2**
(43) Date of publication of application: **19.04.2017**
(21) Application number: 16185926.9
(22) Date of filing: 26.08.2016
(51) Int. Cl.: A61F 2/44

(54) **INTERVERTEBRAL FUSION APPARATUS FOR POSITIONING AND COMBINING VERTEBRAE**

(30) Priority: 26.08.2015 US 201562210048 P
(71) Applicant: Wiltrom Co., Ltd., Hsinchu County 30261 (TW)
(72) Inventor: Liang, Huang-Chien, 300 Hsinchu City (TW); Su, Yi-Chun, 330 Taoyuan City (TW); Tai, Hung-Yin, 300 Hsinchu City (TW); Chen, Huang-Chi, 310 Hsinchu County (TW)
(74) Representative: Lang, Christian

(57) **Abstract**

The present invention provides an intervertebral fusion apparatus for positioning and combining vertebrae, which uses a solidifiable material and an intervertebral fusion cage concurrently. By diffusing the solidifiable material from the top or bottom surface of the intervertebral fusion cage, the contact area with vertebrae and hence the stability can be increased. In addition, the intervertebral fusion cage provided by the present invention can further include a wing member connected pivotally on the side and operated concurrently with the solidifiable material to change the shape and thus avoid slipping off the operation opening. By using the intervertebral fusion apparatus, the stability of the intervertebral fusion cage during and after surgeries can be improved effectively. Thereby, the success rate and safety of vertebral fusion surgeries can be increased.

## Description

### FIELD OF THE INVENTION

The present invention relates generally to a spine surgery apparatus, and in particular to an intervertebral fusion apparatus for positioning and combining vertebrae.

### BACKGROUND OF THE INVENTION

As people age, degeneration of hard and soft tissues inside the body is unavoidable. Aging of different parts in the body results in a significantly different influence on daily living. In particular, pain in the low back induced by the aging of intervertebral discs has become one of the most annoying symptoms.

Intervertebral discs are connective tissues located between two vertebrae and act as buffers therebetween for easing the tension and pressure generated while vertebrae move and thus avoiding damages and pains on the spine due to excessively strong impacts. An intervertebral disc is normally formed by two parts. The central part is called the nucleus, which mainly provides the shock absorption capability for the intervertebral disc. The ligament attached to the adjacent vertebra and surrounding the nucleus is called the annulus. When the nucleus is dehydrated due to the loss of the ability of absorbing water, it loses its shock absorption capability. Then, the pressure generated by human activities will act directly on the bone structure of the spine. By destroying the structure of the annulus, the support and structure of the whole intervertebral disc will be weakened, which will finally lead to direct compression on vertebrae and generates mechanical low back pains. Even worse, aging of intervertebral discs might narrow the neural foramens surrounding the intervertebral discs or loosen the ligaments surrounding the spinal canal. Thereby, the nerve roots will be compressed what can result in serious neuropathic pains. Both will make patients uncomfortable.

Currently, the surgery for treating the serious degenerative disc disease is mainly the spinal fusion surgery. According to the surgery, the intervertebral discectomy is firstly adopted to take out the degenerated intervertebral disc. Next, a graft is placed inside the space between the two vertebrae after the intervertebral discectomy. Finally, pedicle screw fixators are used for fixing and fusing the vertebrae. The adopted graft can be an allograft donated by others or an autologous graft taken from the patient's own body. The implantation methods include implanting the whole bone by wedging. Alternatively, the autologous graft can be placed in a fusion cage. Then the fusion cage, which acts as a carrier, is placed into the body. Thereby, an effect of stimulating fusion of the two vertebrae can be achieved.

The intervertebral fusion surgery using intervertebral fusion cage has become the mainstream surgery for treating intervertebral disc related diseases for the reason that the weight distribution of vertebrae can be re-balanced because of the immediate recovery of the height of intervertebral discs and of the normal curve of vertebrae. Furthermore, after the angles of vertebrae are recovered, the compression on the neural foramens surrounding intervertebral discs can be reduced, which eases immediately the neuropathic pains caused by nerve compression. In addition, the intervertebral fusion fixator also provides a highly stable environment for fusing two vertebrae with low surgical risk. Given the advantages in efficacy and safety, it naturally becomes the primary choice for treating intervertebral disc related diseases.

Unfortunately, current intervertebral fusion cages still have some drawbacks. First, the contact area between the implanted intervertebral fusion cage and the vertebrae is quite small, which brings about shift in the location of the intervertebral fusion cage owing to unstable friction source during the operation. Although the top and bottom surfaces of some current intervertebral fusion cages are designed zigzag for increasing the friction between the intervertebral fusion cage and the vertebrae, the improvement is still limited. In addition, because the intervertebral fusion cage is implanted into the body through an opening at the intervertebral disc, the height of the intervertebral fusion cage and the size of the opening must be pretty close. Thereby, after the operation for implanting the intervertebral fusion cage is completed, without sufficient fixation, there are some risks that the intervertebral fusion cage will slip off the opening prepared for the intervertebral disc operation. As a less serious consequence, it influences the efficiency of autologous recovery of vertebrae. As a severe one, nerves might be compressed resulting in unexpected harms.

Accordingly, how to provide an intervertebral fusion cage with high operative stability and does not slip off the operation opening for guaranteeing superior surgical quality for intervertebral fusion surgeries has become the major emphasis of the field.

### SUMMARY

An objective of the present invention is to provide an intervertebral fusion apparatus for positioning and combining vertebrae. By operating the intervertebral fusion cage and a solidifiable material concurrently, the solidifiable material can diffuse to the top and bottom surfaces of the intervertebral fusion cage from the accommodating space. Thereby, the contact areas between the intervertebral fusion cage and the top and bottom vertebrae can be increased and thus improving the stability of the intervertebral fusion cage during the surgery process.

Another objective of the present invention is to provide an intervertebral fusion apparatus for positioning and combining vertebrae. By connecting pivotally a wing member to the side of the intervertebral fusion cage and extending the wing member after being implanted into the intervertebral disc location, the intervertebral fusion cage will not slip off the intervertebral disc location after completion of the surgery.

A further objective of the present invention is to provide an intervertebral fusion apparatus for positioning and combining vertebrae. By using the form change provided by the extended wing member and the contacting force provided by the solidifiable material, the intervertebral fusion cage can be combined between two vertebrae more securely.

In order to achieve the above objectives, the present invention discloses an intervertebral fusion apparatus for positioning and combining vertebrae placed between a top vertebra and a bottom vertebra. The structure of the intervertebral fusion apparatus comprises an intervertebral fusion cage including an inlet on the front surface and an accommodating space therein. The inlet communicates with the accommodating space. In addition, the intervertebral fusion apparatus further comprises a solidifiable material injected from the inlet of the intervertebral fusion cage, and entering and filling the accommodating space.

According to an embodiment of the present invention, the intervertebral fusion cage comprises a top penetrating hole and a bottom penetrating hole in at least the vertical direction. The top and bottom penetrating hole each communicates with the accommodating space. Besides, the top and bottom penetrating holes include a top accommodating space and a bottom accommodating space, respectively. The solidifiable material passes through the accommodating space and enters and fills the top or bottom accommodating space. The solidifiable material entering and filling the top or bottom accommodating space will contact the top or bottom vertebra.

According to an embodiment, the intervertebral fusion cage includes one or more side penetrating holes. A wing member is pivotably disposed on the intervertebral fusion cage and is located in the side penetrating hole.

According to an embodiment of the present invention, the wing member is pushed by the solidifiable material injected into the accommodating space to rotate pivotally and is thus extended to the side.

According to an embodiment of the present invention, the wing member pivots on the front edge of the side penetrating hole correspondingly.

According to an embodiment of the present invention, the solidifiable material is selected from the group consisting of amorphous calcium phosphate, poorly crystalline calcium phosphate, hydroxyapatite, carbonated apatite (calcium-deficient hydroxyapatite), monocalcium phosphate, calcium metaphosphate, heptacalcium phosphate, dicalcium phosphate dihydrate, tetracalcium phosphate, octacalcium phosphate, calcium pyrophosphate, tricalcium phosphate, calcium sulfate hemihydrate, calcium sulfate dihydrate, polysaccharides, nucleic acids, carbohydrates, proteins, polypeptides, poly(α-hydroxy acids), poly(lactones), poly(amino acids), poly(anhydrides), poly(orthoesters), poly(anhydride-co-imides), poly(orthocarbonates), poly(α-hydroxy alkanoates), poly(dioxanones), poly(phosphoesters), poly(L-lactide) (PLLA), poly(D,L-lactide) (PDLLA), polyglycolide (PGA), poly(lactide-co-glycolide (PLGA), poly(L-lactide-co-D, L-lactide), poly(D,L-lactide-co-trimethylene carbonate), polyhydroxybutyrate (PHB), poly(ε-caprolactone), poly(ε-valerolactone), poly(γ-butyrolactone), poly(caprolactone), polyacrylic acid, polycarboxylic acid, poly(allylamine hydrochloride), poly(diallyldimethylammonium chloride), poly(ethyleneimine), polypropylene fumarate, polyvinyl alcohol, polyvinylpyrrolidone, polyethylene, polymethylmethacrylate, carbon fibers, poly(ethylene glycol), poly(ethylene oxide), poly(vinyl alcohol), poly(vinylpymolidone), poly(ethyloxazoline), poly(ethylene oxide)-co-poly(propylene oxide) block copolymers, poly(ethylene terephthalate)polyamide and copolymers thereof, alginic acid, arabic gum, guar gum, xantham gum, gelatin, chitin, chitosan, chitosan acetate, chitosan lactate, chondroitin sulfate, N,O-carboxymethyl chitosan, dextran (e.g., α-cyclodextrin, β-cyclodextrin, γ-cyclodextrin, or sodium dextran sulfate), fibrin glue, glycerol, hyaluronic acid, sodium hyaluronate, cellulose (e.g., methylcellulose, carboxy methylcellulose, hydroxypropyl methylcellulose, or hydroxyethyl cellulose), glucosamine, proteoglycan, starch (e.g., hydroxyethyl starch or starch soluble), lactic acid, pluronic, sodium glycerophosphate, collagen, glycogen, keratin, silk fibroin and the combination thereof.

According to an embodiment of the present invention, the solidifiable material is a thermoplastic fluid.

According to an embodiment of the present invention, the inlet of the intervertebral fusion cage includes a connecting device used for connecting with an injector.

Moreover, in order to achieve the above objectives, the present invention also discloses an intervertebral fusion apparatus for positioning and combining vertebrae comprising an intervertebral fusion cage, which includes an inlet on the front surface and an accommodating space therein. The inlet communicates with the accommodating space. In addition, the intervertebral fusion cage includes a side penetrating hole in at least the lateral direction. The side penetrating hole communicates with the accommodating space. Besides, the intervertebral fusion apparatus comprises one or more wing member pivoting on the intervertebral fusion cage and located in the side penetrating hole, and thus enabling the wing member to have contacting effects and connecting effects by extending outwards.

According to an embodiment of the present invention, the accommodating space further includes a solidifiable material filling to the top or bottom accommodating space.

According to an embodiment of the present invention, the wing member is pushed by the solidifiable material injected into the accommodating space to rotate pivotally and is thus extended to the side.

### BRIEF DESCRIPTION OF THE DRAWINGS

Figures 1A and 1B show a top view and a right-side view of the intervertebral fusion apparatus for positioning and combining vertebrae in the operational state according to a preferred embodiment of the present invention;
Figures 2A and 2B show side views of the top and bottom surfaces of the intervertebral fusion cage of the intervertebral fusion apparatus according to a preferred embodiment of the present invention;
Figure 3 shows a side view of the intervertebral fusion cage combined with the operational device according to a preferred embodiment of the present invention;
Figure 4 shows a side view of the intervertebral fusion cage according to another preferred embodiment of the present invention;
Figure 5 shows a side view of the intervertebral fusion cage according to another preferred embodiment of the present invention; and
Figure 6 shows a top view of the intervertebral fusion apparatus for positioning and combining vertebrae in the operational state according to another preferred embodiment of the present invention.

### DETAILED DESCRIPTION

In order to make the structure and characteristics as well as the effectiveness of the present invention to be further understood and recognized, a detailed description of the embodiments of the present invention along with accompanying figures is provided as follows.

According to the present invention, a novel intervertebral fusion apparatus for positioning and combining vertebrae and the method for the use thereof are provided for solving the problems of shift of the intervertebral fusion cage from the surgical site due to an unstable friction source and further solving the problem of the intervertebral fusion cage slipping off the operation opening easily after completion of the surgery. The intervertebral fusion apparatus for positioning and combining vertebrae adopts an intervertebral fusion cage and a filling material to operate concurrently. The intervertebral fusion cage includes a central accommodating space communicating with a top opening and a bottom opening. After the intervertebral fusion cage is set up in a target positioning region during the spine surgery process, the filling material is delivered to the target positioning region from the accommodating space and the top and bottom openings of the intervertebral fusion cage for increasing the junction area with the surrounding environment. By using the limiting property of the filling material, the overall stability of the intervertebral fusion apparatus can be improved. Thereby, the problem of shift of the general intervertebral fusion apparatus from the surgical site due to unstable friction sources can be overcome. In addition, the intervertebral fusion cage according to the present invention further comprises one or more side openings pivoted by a wing member. As the filling material is pushed outwards from the one or more side openings, the wing member can be extended. And hence the area of the overall intervertebral fusion apparatus can be increased thereby for overcoming the problem of the intervertebral fusion cage slipping off the operation opening easily after completion of the surgery.

In the following, the components, properties and the disposal of the intervertebral fusion apparatus for positioning and combining vertebrae according to the present invention will be described.

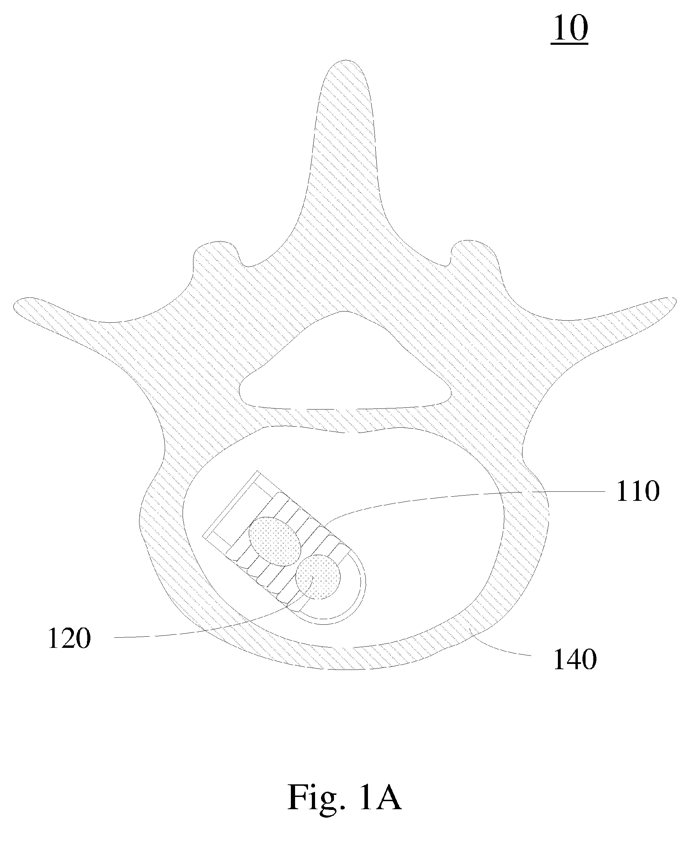

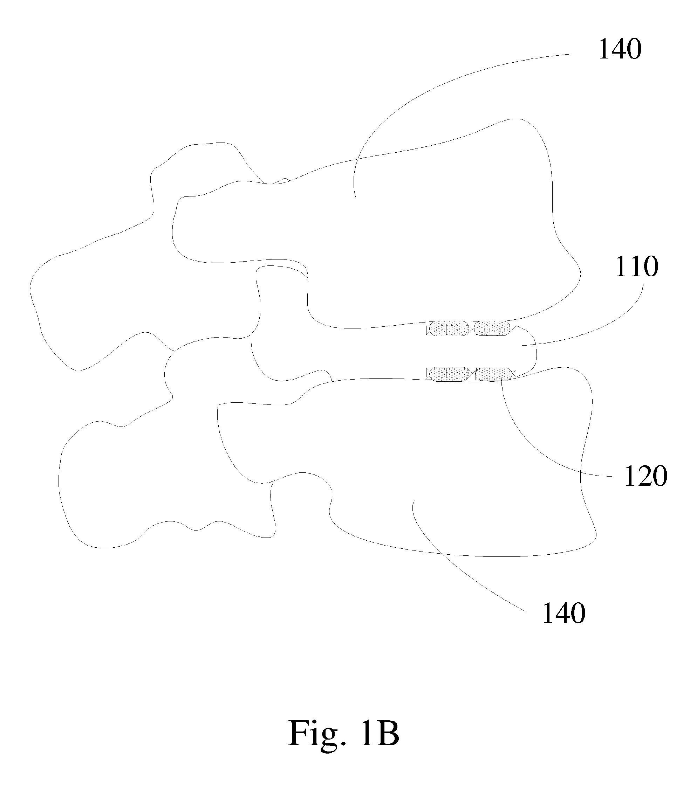

Please refer to Figures 1A and 1B, which show a top view and a right-side view of the intervertebral fusion apparatus for positioning and combining vertebrae in the operational state according to a preferred embodiment of the present invention. As shown in the figures, the intervertebral fusion apparatus for positioning and combining vertebrae 10 provided by the present invention is disposed between two vertebrae and comprises an intervertebral fusion cage 110 and a filling material 120. By using the contact area for the connection with the top and bottom vertebrae provided by the intervertebral fusion cage 110 and the filling material 120, the friction and stability required for positioning between and combining with adjacent vertebrae can be provided by the intervertebral fusion apparatus for positioning and combining vertebrae 10.

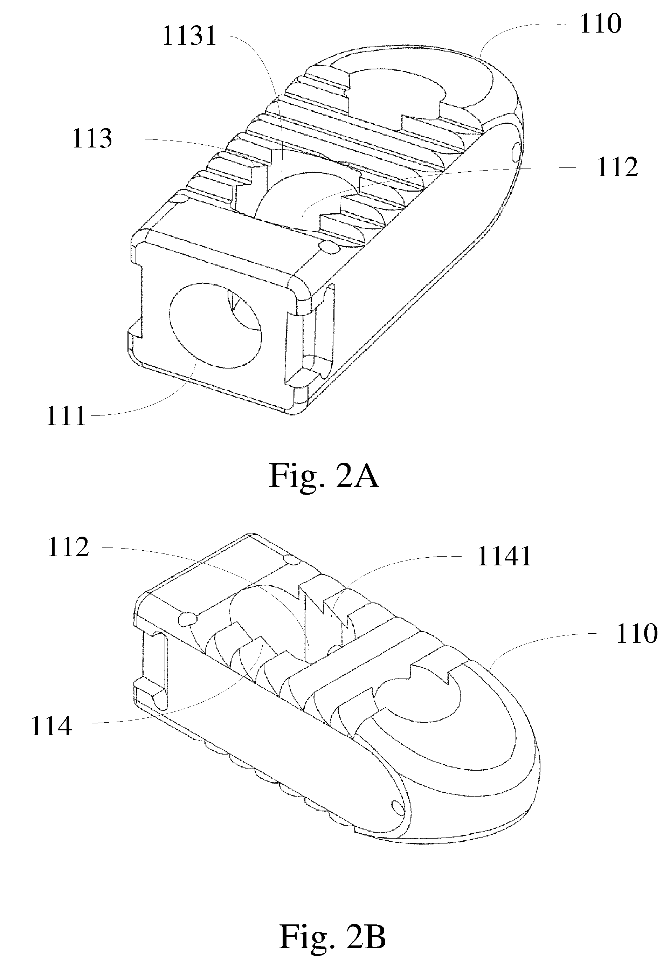

Next, please refer to Figures 2A and 2B, which show side views of the top and bottom surfaces of the intervertebral fusion cage of the intervertebral fusion apparatus according to a preferred embodiment of the present invention. The structure of the intervertebral fusion cage 110 of the intervertebral fusion apparatus for positioning and combining vertebrae 10 comprises an inlet 111 on the front end surface, an accommodating space 112 inside the intervertebral fusion cage 110, and one or more penetrating holes in the vertical direction. The one or more penetrating holes can be a top penetrating hole 113 or a bottom penetrating hole 114. The top penetrating hole 113 includes a top penetrating accommodating space 1131. The top penetrating hole 113 includes a bottom penetrating accommodating space 1141. Besides, the inlet 111 and the top penetrating accommodating space 1131 or the bottom penetrating accommodating space 1141 communicates with the accommodating space 112, respectively.

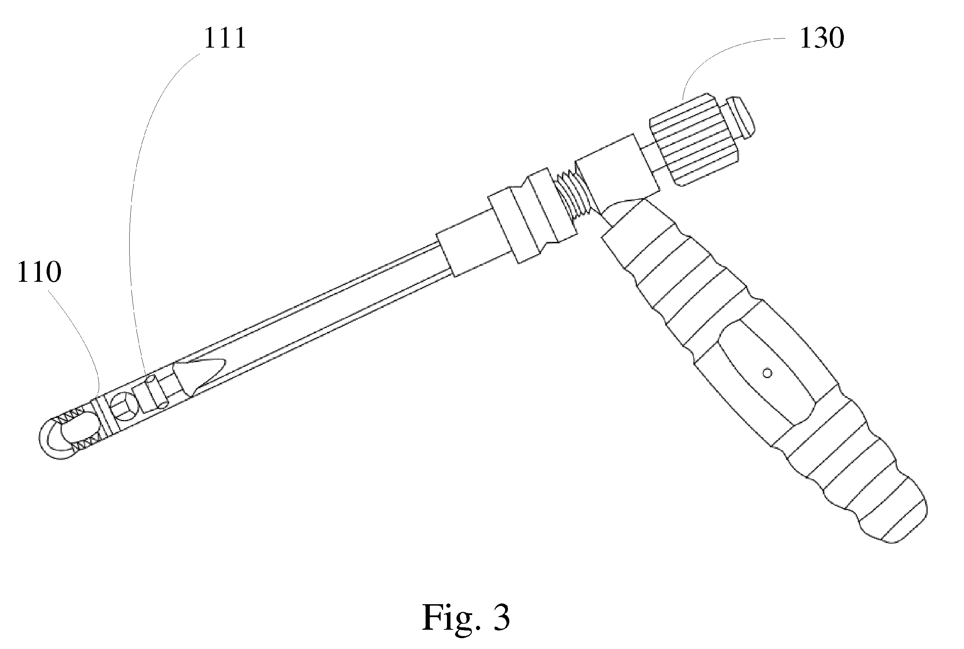

Next, please refer to Figure 3, which shows a side view of the intervertebral fusion cage combined with the operational device according to a preferred embodiment of the present invention. As shown in the figure, according to the intervertebral fusion cage 110 provided by the present invention, the inlet 111 is not only the entrance for the filling material 120 to enter the intervertebral fusion cage 110. It can be further combined with different operative apparatuses for achieving the purposes of positioning and combining vertebrae by the use of the intervertebral fusion apparatus 10 provided by the present invention. For example, the intervertebral fusion cage 110 can be combined with an implant device 130 through the inlet 111. Then the implant device 130 can deliver the intervertebral fusion cage 110 to the affected part from the outside of the patient's body. By further placing the intervertebral fusion cage 110 by striking, the purpose of delivering the intervertebral fusion cage 110 to the target region can be achieved. In addition, the intervertebral fusion cage 110 can be combined with a filling-material injector 131 through the inlet 111. The filling-material injector 131 can deliver the filling material 120 to the intervertebral fusion cage 110 through the inlet 111, and fill and diffuse the filling material 120 following the filling direction as described above for achieving the purpose of delivering the filling material 120 to the affected part and positioning and combining vertebrae. Based on the above principles, the inlet 111 provided by the present invention can be combined with various operative apparatuses using tight, screw, or wedge connecting methods. Furthermore, the inlet 111 provided by the present invention includes a connecting device used for the connection with the filling-material injector 131 or the implant device 130.

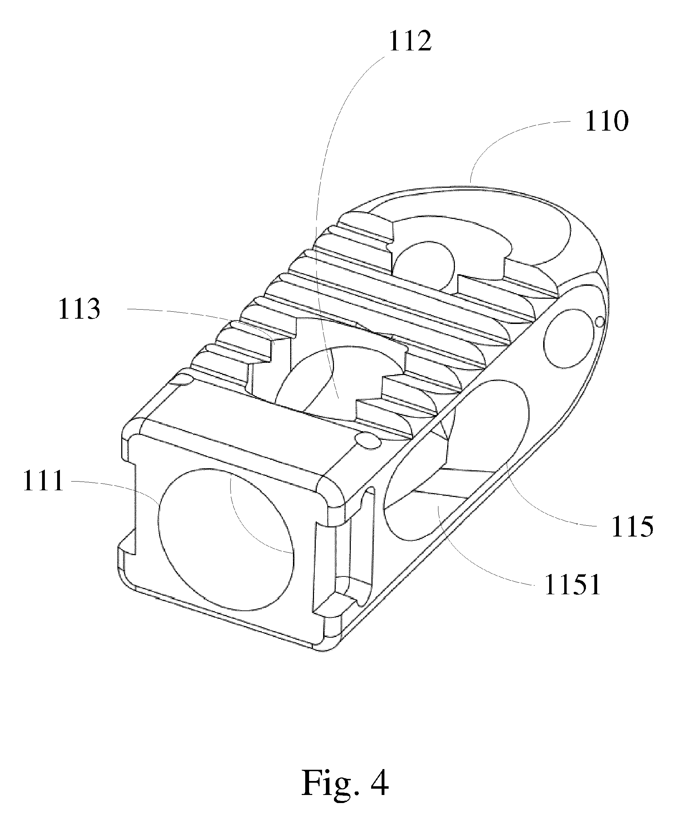

Next, please refer to Figure 4, which shows a side view of the intervertebral fusion cage according to another preferred embodiment of the present invention. As shown in the figure, the intervertebral fusion cage 110 provided by the present invention can further include one or more side penetrating holes 115, which include a side accommodating space 1151 communicating, likewise, with the accommodating space 112. By disposing the one or more side penetrating holes 115 and the side accommodating space 1151, the filling material 120 entering the accommodating space 112 from the inlet 111 can diffuse and flow towards the one or more side penetrating holes 115. As the filling material 120 is filled to the side of the intervertebral fusion cage 110 and limits the movable range of the intervertebral fusion cage 110, the lateral width of the intervertebral fusion apparatus for positioning and combining vertebrae 10 provided by the present invention can be extended effectively, which makes the overall lateral width of the intervertebral fusion apparatus 10 greater than the lateral width of the operation opening for implanting the intervertebral fusion apparatus 10. Consequently, the problem that the intervertebral fusion cage can slip off the operation opening after completion of the surgery can be avoided effectively.

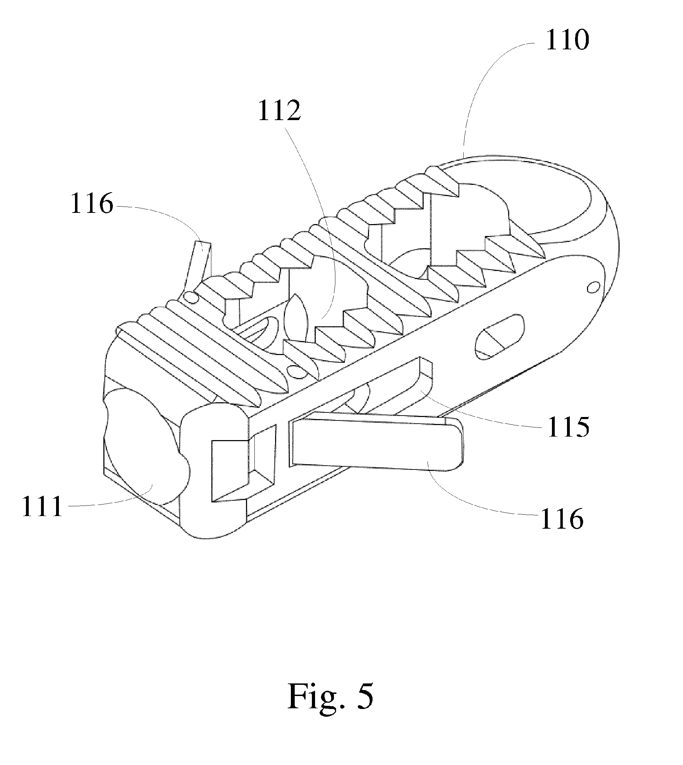

Next, please refer to Figure 5, which shows a side view of the intervertebral fusion cage according to another preferred embodiment of the present invention. As shown in the figure, the intervertebral fusion cage 110 provided by the present invention further includes a wing member 116 pivoted on the intervertebral fusion cage 110 and disposed in the one or more side penetrating hole 115. Based on the above properties, when the filling material 120 enters the accommodating space 112 and is guided by the one or more side penetrating holes 115 to diffuse and flow to the side of the intervertebral cage 110, the wing member 116 will extend pivotally to the side as pushed by the filling material 120. The extended wing can increase the lateral width of the intervertebral fusion apparatus for positioning and combining vertebrae 10 as provided by the present invention. In addition, the extended wing member 116 can also limit the flowing direction of the filling material 120. Thereby, the wing member 116 can not only achieve the effect of avoiding slipping off the operation opening but also can control the range of limiting the intervertebral fusion cage 110 by the filling material 120 flowing in the spinal canal.

According to a preferred embodiment of the present invention, the wing member 116 pivots on the front edge of the side penetrating hole 115, namely, the front location of the intervertebral fusion cage 110, and is on the same side of the inlet 111. Based on the above design, when the wing member 116 pivots to the side and extends as pushed by the filling material 120, the extended wing can avoid effectively that the spinal fusion apparatus gets slipped off the operation opening. In addition, it also can prevent the filling material 120 from spilling to the other tissues of the body, which might result in unnecessary surgical danger to the patient.

The filling material 120 of the intervertebral fusion apparatus for positioning and combining vertebrae 10 according to the present invention is a fluid material. The fluid material is not limited to the fluid form. Any material that is distributive and able to flow to the accommodating space 112, the top accommodating space 1131, the bottom accommodating space 1141 and the side accommodating space 1151 can be used as the filling material 120 according to the present invention. Based on the above description, the filling material 120 according to the present invention can be filling particles, a solidifiable material or a thermoplastic fluid.

The filling particles provided by the present invention are highly fluid and distributive for being distributed into the intervertebral fusion cage and the spinal canal. By filling completely the space inside the intervertebral fusion cage and the spinal canal, the intervertebral fusion apparatus for positioning and combining vertebrae is left with no space to move and hence can be fixed inside the spinal canal. Based on the above properties, the filling particles provided by the present invention can be ceramic particles.

The solidifiable material provided by the present invention is a fluid. It is a material starting the solidification reaction by time or temperature changes. Based on the above principle, the solidifiable material provided by the present invention is in a fluid form. The fluid can be, but is not limited to, plastics or polymer solutions in the liquid, gel, or melt state. Besides, in order to achieve the purpose of starting the solidification reaction by time or temperature changes as described above, the fluid provided by the present invention is preferably a thermoplastic fluid or a fluid doped with a curing agent. Based on the above principles, the filling material 120 is selected from a group consisting of amorphous calcium phosphate, poorly crystalline calcium phosphate, hydroxyapatite, carbonated apatite (calcium-deficient hydroxyapatite), monocalcium phosphate, calcium metaphosphate, heptacalcium phosphate, dicalcium phosphate dihydrate, tetracalcium phosphate, octacalcium phosphate, calcium pyrophosphate, tricalcium phosphate, calcium sulfate hemihydrate, calcium sulfate dihydrate, polysaccharides, nucleic acids, carbohydrates, proteins, polypeptides, poly(α-hydroxy acids), poly(lactones), poly(amino acids), poly(anhydrides), poly(orthoesters), poly(anhydride-co-imides), poly(orthocarbonates), poly(α-hydroxy alkanoates), poly(dioxanones), poly(phosphoesters), poly(L-lactide) (PLLA), poly(D,L-lactide) (PDLLA), polyglycolide (PGA), poly(lactide-co-glycolide (PLGA), poly(L-lactide-co-D, L-lactide), poly(D,L-lactide-co-trimethylene carbonate), polyhydroxybutyrate (PHB), poly(ε-caprolactone), poly(ε-valerolactone), poly(γ-butyrolactone),poly(caprolactone), polyacrylic acid, polycarboxylic acid, poly(allylamine hydrochloride), poly(diallyldimethylammonium chloride), poly(ethyleneimine), polypropylene fumarate, polyvinyl alcohol, polyvinylpyrrolidone, polyethylene, polymethylmethacrylate, carbon fibers, poly(ethylene glycol), poly(ethylene oxide), poly(vinyl alcohol), poly(vinylpyrrolidone), poly(ethyloxazoline), poly(ethylene oxide)-co-poly(propylene oxide) block copolymers, poly(ethylene terephthalate)polyamide and copolymers thereof, alginic acid, arabic gum, guar gum, xantham gum, gelatin, chitin, chitosan, chitosan acetate, chitosan lactate, chondroitin sulfate, N,O-carboxymethyl chitosan, dextran (e.g., α-cyclodextrin, β-cyclodextrin, γ-cyclodextrin, or sodium dextran sulfate), fibrin glue, glycerol, hyaluronic acid, sodium hyaluronate, cellulose (e.g., methylcellulose, carboxy methylcellulose, hydroxypropyl methylcellulose, or hydroxyethyl cellulose), glucosamine, proteoglycan, starch (e.g., hydroxyethyl starch or starch soluble), lactic acid, pluronic, sodium glycerophosphate, collagen, glycogen, keratin, silk fibroin and the combination thereof. Preferably, the solidifiable material provided by the present invention is a bioabsorbable material or a biodegradable material.

In the following, please refer to Figures 1A, 1B, and 2. They are used for describing the relations among the components of the intervertebral fusion apparatus for positioning and combining vertebrae 10 of the present invention. As shown in the figures, according to the intervertebral fusion apparatus for positioning and combining vertebrae 10 of the present invention, the filling material 120 is injected from the inlet 111 located at the front of the intervertebral fusion cage 110. Afterwards, the filling material 120 enters the accommodating space 112 communicating with the inlet 111 and starts filling. Meanwhile, during the process of the filling material 120 filling the accommodating space 112 or after the accommodating space 112 is completely filled, a part of the filling material 120 will flow and diffuse to the top or bottom accommodating space 1131, 1141 communicating with the accommodating space 112, and then spill out of the intervertebral fusion cage 110 from the top or bottom penetrating hole 113, 114. At this time, the spilled filling material 120 will contact a vertebra 140 for increasing the contact area between the intervertebral fusion cage 110 and the adjacent vertebra 140. Besides, when the spilled filling material 120 is increased, the filling material 120 will extend to the front end and rear end of the intervertebral fusion cage 110 along a top surface 117 and a bottom surface 118 for providing more contact surface area. By means of the above process, the intervertebral fusion apparatus of the present invention can improve the stability and thus presents the intervertebral fusion apparatus for positioning and combining vertebrae 10 according to the first embodiment of the present invention.

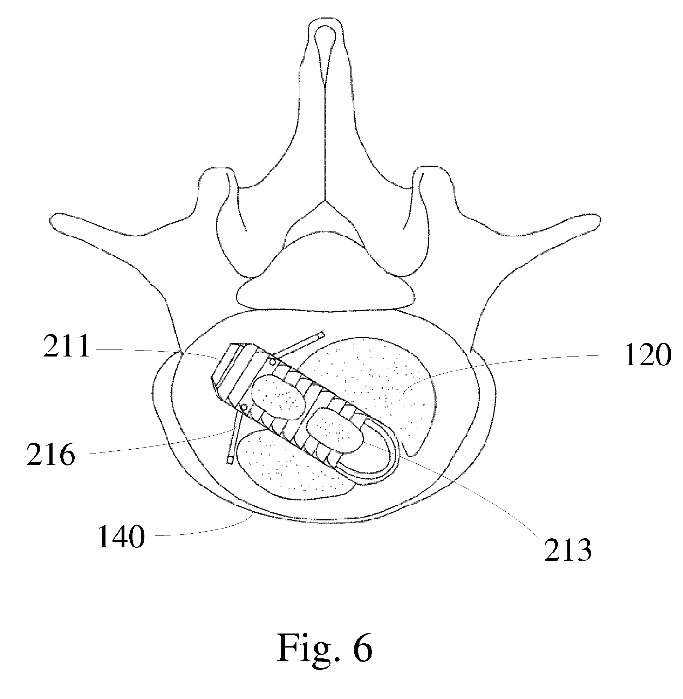

Next, please refer to Figure 5 and Figure 6. Figure 6 shows a top view of the intervertebral fusion apparatus for positioning and combining vertebrae in the operational state according to another preferred embodiment of the present invention. As shown in the figures, the present provides an intervertebral fusion apparatus 20 in another form disposed between two vertebrae. The structure of the intervertebral fusion apparatus 20 comprises an intervertebral fusion cage 210 and one or more wing members 216 pivoted on the intervertebral fusion cage 210. The intervertebral fusion cage 20 includes an inlet 211 on the front end surface and an accommodating space 212 therein. The inlet 211 communicates with the accommodating space 212. The intervertebral fusion cage 210 includes one or more side penetrating holes 215 in the lateral direction communicating with the accommodating space 212. In addition, the one or more wing members 216 pivot on the intervertebral fusion cage 20 and are disposed in the one or more side penetrating holes 215, such that the one or more wing members 216 can extend outwards, facilitating contacting effects and connecting effects. Thereby, when the intervertebral fusion apparatus 20 is positioned, an external force or an inertial force can be exerted on the intervertebral fusion apparatus 20 to deform the one or more wing members 216. The deformed one or more wing members 216 will contact the sidewall of the vertebra 140 or will be against the sidewall of the vertebra 140 for producing friction or resistance and thus achieving the effect of stabilizing the intervertebral fusion apparatus 20 inside vertebrae. Based on the above principles, the one or more wing members 216 provided by the present invention are not limited to pivot on the front end or rear end of the intervertebral fusion cage 210. That is to say, the one or more wing members 216 are not limited to pivot on the front edge or rear edge of the one ore more side penetrating holes 215.

According to the intervertebral fusion apparatus 20 as described above, the intervertebral fusion cage 210 can further include a top penetrating hole 213 and a bottom penetrating hole 214 communicating with the accommodating space 212. The top and bottom penetrating holes 213, 214 include a top accommodating space 2131 and a bottom accommodating space 2141, respectively, and are operated along with the filling material 120, such that the wing member 216 can be pushed and pivoted by the filling material 120 and thus extending to the side of the intervertebral fusion cage 210. The relative positions of components, the operational direction of components, and the achieved effects of the intervertebral fusion apparatus 20 operating concurrently with the filling material 120 are identical to those of the intervertebral fusion apparatus 10. Hence, the details will not be described herein again.

To sum up, the present invention indeed provides an intervertebral fusion apparatus for positioning and combining vertebrae. It can overcome the shortcomings of instability during implanting and can overcome the problem of slipping off the intervertebral disc easily after implantation due to insufficient contact area between the intervertebral fusion cage and vertebrae. According to the present invention, by using a solidifiable material and providing the top and bottom penetrating holes in the intervertebral fusion cage, the solidifiable material can be added continuously for contacting the top and bottom vertebrae and hence an increase of the contact area, friction and adhesion between the overall structure and the vertebrae can be achieved. Consequently, the required stability during the early stage of a surgery for implanting the intervertebral fusion apparatus for positioning and combining vertebrae can be improved effectively. In addition, by using the design of the side penetrating hole and the wing member, the overall lateral width of the intervertebral fusion apparatus is increased and thus slipping off the surgery site after completion of surgery can be effectively avoided. The design can further control the filling direction of the solidifiable material as used concurrently with the solidifiable material and thus can prevent the solidifiable to get in contact with other human tissues than at the surgery site, which might lead to unnecessary harms. Thereby, the present invention indeed provides an intervertebral fusion apparatus for positioning and combining vertebrae having high operational stability and safety and improves the technical standard of the related field.

Accordingly, the present invention conforms to the legal requirements owing to its novelty, nonobviousness, and utility. However, the foregoing description is only for illustrating embodiments of the present invention and is not used to limit the scope and range of the present invention. Those equivalent changes or modifications made according to the shape, structure, feature or spirit as described in the claims of the present invention are included in the appended claims of the present invention.

## Claims

1. An intervertebral fusion apparatus for positioning and combining vertebrae, that is disposable between two vertebrae and comprises:
an intervertebral fusion cage, including an inlet on the front surface, an accommodating space, and a top penetrating hole and a bottom penetrating hole in the vertical direction, wherein said inlet, said top penetrating hole, and said bottom penetrating hole communicate with said accommodating space, and wherein said top penetrating hole and said bottom penetrating hole include a top accommodating space and a bottom accommodating space, respectively; and
a filling material, injected through said inlet for entering said accommodating space and flowing to said top accommodating space and said bottom accommodating space.

2. The intervertebral fusion apparatus for positioning and combining vertebrae of claim 1, wherein said inlet of said intervertebral fusion cage includes a connecting device used for the connection with an injector.

3. The intervertebral fusion apparatus for positioning and combining vertebrae of claim 1, wherein said filling material can enter and fill said top accommodating space or said bottom accommodating space through said accommodating space, so that said filling material entering and filling said top accommodating space or said bottom accommodating space gets in contact with a top vertebra or a bottom vertebra.

4. The intervertebral fusion apparatus for positioning and combining vertebrae of claim 1, wherein said intervertebral fusion cage includes one or more side holes having a side accommodating space communicating with said accommodating space.

5. The intervertebral fusion apparatus for positioning and combining vertebrae of claim 4, wherein said intervertebral fusion cage includes a wing member pivoted on said intervertebral fusion cage and located outside of said one or more side holes, wherein said wing member can be pushed by said filling material injected into said accommodating space and is pivotally extended to the side.

6. The intervertebral fusion apparatus for positioning and combining vertebrae of claim 5, wherein said wing member is pivoted on the front edge of said intervertebral fusion cage.

7. The intervertebral fusion apparatus for positioning and combining vertebrae of claim 1, wherein said filling material includes filling particles, a solidifiable material or a thermoplastic fluid.

8. The intervertebral fusion apparatus for positioning and combining vertebrae of claim 1, wherein said solidifiable material is selected from a group consisting of amorphous calcium phosphate, poorly crystalline calcium phosphate, hydroxyapatite, carbonated apatite (calcium-deficient hydroxyapatite), monocalcium phosphate, calcium metaphosphate, heptacalcium phosphate, dicalcium phosphate dihydrate, tetracalcium phosphate, octacalcium phosphate, calcium pyrophosphate, tricalcium phosphate, calcium sulfate hemihydrate, calcium sulfate dihydrate, polysaccharides, nucleic acids, carbohydrates, proteins, polypeptides, poly(α-hydroxy acids), poly(lactones), poly(amino acids), poly(anhydrides), poly(orthoesters), poly(anhydride-co-imides), poly(orthocarbonates), poly(α-hydroxy alkanoates), poly(dioxanones), poly(phosphoesters), poly(L-lactide) (PLLA), poly(D,L-lactide) (PDLLA), polyglycolide (PGA), poly(lactide-co-glycolide (PLGA), poly(L-lactide-co-D, L-lactide), poly(D,L-lactide-co-trimethylene carbonate), polyhydroxybutyrate (PHB), poly(ε-caprolactone), poly(ε-valerolactone), poly(γ-butyrolactone), poly(caprolactone), polyacrylic acid, polycarboxylic acid, poly(allylamine hydrochloride), poly(diallyldimethylammonium chloride), poly(ethyleneimine), polypropylene fumarate, polyvinyl alcohol, polyvinylpyrrolidone, polyethylene, polymethylmethacrylate, carbon fibers, poly(ethylene glycol), poly(ethylene oxide), poly(vinyl alcohol), poly(vinylpyrrolidone), poly(ethyloxazoline), poly(ethylene oxide)-co-poly(propylene oxide) block copolymers, poly(ethylene terephthalate)polyamide, alginic acid, arabic gum, guar gum, xantham gum, gelatin, chitin, chitosan, chitosan acetate, chitosan lactate, chondroitin sulfate, N,O-carboxymethyl chitosan, dextran (e.g., α-cyclodextrin, β-cyclodextrin, γ-cyclodextrin, or sodium dextran sulfate), fibrin glue, glycerol, hyaluronic acid, sodium hyaluronate, cellulose (e.g., methylcellulose, carboxy methylcellulose, hydroxypropyl methylcellulose, or hydroxyethyl cellulose), glucosamine, proteoglycan, starch (e.g., hydroxyethyl starch or starch soluble), lactic acid, pluronic, sodium glycerophosphate, collagen, glycogen, keratin, silk fibroin and the combination thereof.

9. An intervertebral fusion apparatus for positioning and combining vertebrae, disposable between two vertebrae and comprising:
an intervertebral fusion cage, including an inlet on the front surface and an accommodating space, said inlet communicating with said accommodating space, including one ore more side holed in the lateral direction, said one or more side holes including a side accommodating space communicating with said accommodating space; and
one or more wing members, pivoted on said intervertebral fusion cage and located outside said side hole, and being outward extendable for contacting and connecting adjacent vertebrae.

10. The intervertebral fusion apparatus for positioning and combining vertebrae of claim 9, wherein said wing members are pivoted on the front edge of said intervertebral fusion cage and are opposite to one another.

11. The intervertebral fusion apparatus for positioning and combining vertebrae of claim 9, wherein said inlet of said intervertebral fusion cage includes a connecting device used for the connection with an injector.

12. The intervertebral fusion apparatus for positioning and combining vertebrae of claim 9, wherein said intervertebral fusion cage includes a top penetrating hole and a bottom penetrating hole, wherein said top penetrating hole and said bottom penetrating hole include a top accommodating space and a bottom accommodating space, respectively, and wherein said top accommodating space and said bottom accommodating space communicate with said accommodating space.

13. The intervertebral fusion apparatus for positioning and combining vertebrae of claim 12, wherein said accommodating space includes a filling material that flows to said top accommodating space, said bottom accommodating space and said side accommodating space for filling, wherein said wing member is pushed by said filling material injected into said accommodating space and flowing to said side accommodating space for filling and extends pivotally to the side.

14. The intervertebral fusion apparatus for positioning and combining vertebrae of claim 13, wherein said filling material includes filling particles, a solidifiable material or a thermoplastic fluid.

15. The intervertebral fusion apparatus for positioning and combining vertebrae of claim 14, wherein said solidifiable material is selected from a group consisting of amorphous calcium phosphate, poorly crystalline calcium phosphate, hydroxyapatite, carbonated apatite (calcium-deficient hydroxyapatite), monocalcium phosphate, calcium metaphosphate, heptacalcium phosphate, dicalcium phosphate dihydrate, tetracalcium phosphate, octacalcium phosphate, calcium pyrophosphate, tricalcium phosphate, calcium sulfate hemihydrate, calcium sulfate dihydrate, polysaccharides, nucleic acids, carbohydrates, proteins, polypeptides, poly(α-hydroxy acids), poly(lactones), poly(amino acids), poly(anhydrides), poly(orthoesters), poly(anhydride-co-imides), poly(orthocarbonates), poly(α-hydroxy alkanoates), poly(dioxanones), poly(phosphoesters), poly(L-lactide) (PLLA), poly(D,L-lactide) (PDLLA), polyglycolide (PGA), poly(lactide-co-glycolide (PLGA), poly(L-lactide-co-D, L-lactide), poly(D,L-lactide-co-trimethylene carbonate), polyhydroxybutyrate (PHB), poly(ε-caprolactone), poly(ε-valerolactone), poly(γ-butyrolactone), poly(caprolactone), polyacrylic acid, polycarboxylic acid, poly(allylamine hydrochloride), poly(diallyldimethylammonium chloride), poly(ethyleneimine), polypropylene fumarate, polyvinyl alcohol, polyvinylpyrrolidone, polyethylene, polymethylmethacrylate, carbon fibers, poly(ethylene glycol), poly(ethylene oxide), poly(vinyl alcohol), poly(vinylpyrrolidone), poly(ethyloxazoline), poly(ethylene oxide)-co-poly(propylene oxide) block copolymers, poly(ethylene terephthalate)polyamide" alginic acid, arabic gum, guar gum, xantham gum, gelatin, chitin, chitosan, chitosan acetate, chitosan lactate, chondroitin sulfate, N,O-carboxymethyl chitosan, dextran (e.g., α-cyclodextrin, β-cyclodextrin, γ-cyclodextrin, or sodium dextran sulfate), fibrin glue, glycerol, hyaluronic acid, sodium hyaluronate, cellulose (e.g., methylcellulose, carboxy methylcellulose, hydroxypropyl methylcellulose, or hydroxyethyl cellulose), glucosamine, proteoglycan, starch (e.g., hydroxyethyl starch or starch soluble), lactic acid, pluronic, sodium glycerophosphate, collagen, glycogen, keratin, silk fibroin and the combination thereof.
